# EUROPEAN PATENT SPECIFICATION

(11) **EP 3 257 439 B1**
(45) Date of publication and mention of the grant of the patent: **18.11.2020**
(21) Application number: 16174202.8
(22) Date of filing: 13.06.2016
(51) Int. Cl.: A61B 5/05, A61B 5/06, A61B 5/103, A61B 5/00

(54) **A TISSUE ANOMALY DETECTION APPARATUS COMPRISING A PROBE TRANSMITTER DEVICE**
GEWEBEANOMALIEDETEKTIONSVORRICHTUNG MIT EINER SONDENSENDERVORRICHTUNG
APPAREIL DE DÉTECTION D'ANOMALIE TISSULAIRE COMPRENANT UN DISPOSITIF ÉMETTEUR DE SONDE

(43) Date of publication of application: 20.12.2017
(73) Proprietor: Medielma S.r.l., 20145 Milano (IT)
(72) Inventor: CANONICO, Michelangelo, 20149 Milano (IT); BELLOROFONTE, Carlo, 20157 Milano (IT)
(74) Representative: Postiglione, Ferruccio

(56) References cited:
- EP-A1- 0 158 690
- EP-A1- 2 465 428
- EP-A1- 2 912 998
- JP-A- H 042 369
- JP-A- 2011 205 537
- JP-A- 2014 153 297
- US-A1- 2005 107 692
- US-A1- 2011 215 983
- US-A1- 2012 150 482

## Description

### Technical field

The present invention relates to electromagnetic detection systems for the detection of anomalies (e.g. tumors and calcifications) in living tissue, and, particularly but not limitatively, to early detection of breast, colorectal and prostatic cancer.

### Background of the invention

Document EP-A-2465428 discloses a tissue anomaly detection method employing a probe transmitter device, i.e. a transmitter equipped with one or two antennas coupled in the near field conditions with tissues. The probe transmitter device described in this prior art document is moved by a clinician for irradiating a tissue of a patient in near-field conditions so as to produce a radio-frequency signal resulting from the interaction with the aforementioned tissue. The system also comprises a receiving device for receiving said resulting signal and a processing module structured to process the received data in order to provide an information indicating the detection of an anomaly in said tissue. In particular, the probe transmitter device has a fixed radiation frequency range and it is structured so that the resulting signal is a scattered signal resulting from a combination of the incident signal and an induced radio-frequency signal resulting from the coupled tissue.

According the description of EP-A-2465428 the scattered signal is received and electronically analysed to detect an anomaly associated with nulls or minima in the pattern of the scattered electrical field power versus frequency and receiving antenna position.

Document EP-A-2912998 discloses a tissue anomaly detection apparatus having a patient positioning structure configured to allow the patient assuming a detection position and a probe antenna device structured to radiate an incident radio-frequency signal. The probe antenna device of this document is mechanically coupled with a motor so as to cause a sliding motion of the probe antenna device.

The Applicant has experimentally noticed that the apparatus described by document EP-A-2912998 does not provide satisfying results with reference to the detection capability.

### Summary of invention

The Applicant has faced the problem of providing an anomaly detection apparatus that allows an efficient detection of the tissue anomalies without requiring an extreme structural and circuital complexity of the apparatus.

The Applicant has found that an enhancing of the anomalies detectability, avoiding excessive complexity, can be obtained with a tissue anomaly detection apparatus as described by the attached claim 1. Particular embodiments of the apparatus are described by the dependent claims 2-18.

### Brief description of the drawings

Further characteristics and advantages will be more apparent from the following description of preferred embodiments given as a way of an example with reference to the enclosed drawings in which:
- figure 1 schematically shows a preferred embodiment of a tissue anomaly detection apparatus;
- figure 1A shows a particular embodiment of a receiving module employable in said anomaly detection apparatus;
- figure 2 shows an embodiment of a probe transmitter device employable in said detection apparatus;
- figure 3 shows by functional modules components included into said probe transmitter device
- figure 4 illustrates a particular embodiment of an antenna radiating element and a respective supporting structure employable in said probe transmitter device.

### Detailed description

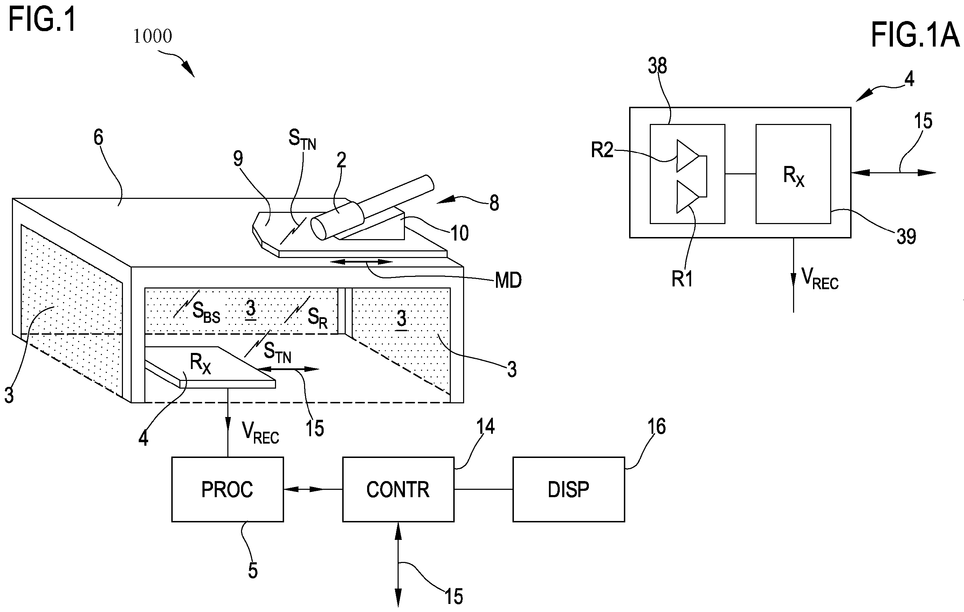

Figure 1 shows an embodiment of a tissue anomaly detection apparatus 1000 which can be used to detect anomalies in tissues of human or animal bodies. The anomalies can be, as an example, tumors and/or calcifications. Particularly, tumors in early stage (e.g. malignant breast, prostatic and colorectal tumors) can be detected by the detection apparatus 1000.

The detection apparatus 1000 comprises: a patient positioning structure 1 configured to allow the patient assuming a detection position, a probe transmitter device 2, a receiving device 4 and a processing module 5 (PROC). Preferably, the detection apparatus 1000 may comprise one or more reflecting walls 3.

Moreover, according to a particular embodiment, the detection apparatus 1000 comprises a controller module 14 (CONTR) connected, via a wired or wireless link 15, to the probe transmitter device 2 and also connected to the processing unit 5, and a display 16 (DISP) .

The patient positioning structure 1 can be a support frame designed to support the patient or one of her/his body part. The example shown in figure 1 refers to a patient positioning structure 1 which can be used to the diagnosis of prostatic cancer, cervical cancer or rectum cancer.

In accordance with an example, which is also suitable for the diagnosis of prostatic cancer, the patient positioning structure 1 comprises a bed type support 6.

The detection apparatus 1000 is also provided with a mounting frame 8 configured to support the probe transmitter device 2. Particularly, the mounting frame 8 is provided with sliding means configured to allow a sliding motion of the probe transmitter device 2 according to, as an example, a direction MD which lies on a plane including the probe axis. This sliding motion allows bringing the probe transmitter device 2 in contact with the patient body, in an operation configuration, or moving the probe transmitter device 2 away from the patient body. Preferably, the sliding means include a base 9, fixed to the bed type support 6, and a slide 10 on which the probe transmitter device 2 is mounted (e.g. in a tilted configuration with respect the bed type support 6). The patient may lie down on the bed type support 6 so as to be in close contact with an external portion of probe transmitter device 2.

According to another embodiment, the probe transmitter device 2 is supported by an articulated arm (not shown) mounted on a frame which can be placed on cart or a tripod. This embodiment is particularly suitable for the diagnosis of anomalies different from the prostatic cancer.

The probe transmitter device 2 is structured to radiate a narrowband incident radio-frequency signal S_{RAD} in order irradiate a patient tissue in near-field conditions.

To the purpose of the present description, a narrowband signal shows a bandwidth Bw which is small enough to use the assumption that a response as result of the interaction with body can be considered constant within the bandwidth Bw, provided that 1/Bw is below the relaxation times of the irradiated patient biological tissues (typical value Bw>1KHz).

The probe transmitter device 2 generates an electromagnetic field (i.e. an incident signal) that locally couples in near-field with the tissues surrounding the contact point. As an example, with reference to the prostatic tumor it is observed that around the perineum the area is electromagnetically very heterogeneous and it cannot be described by just simple macroscopic relative permittivity values. However, for the sake of reasoning, by assuming a relative permittivity at 434 MHz that ranges between 7 and 12 as for mixed fat and bones, the wavelength in the body is about 19-25 cm. Prostate size is 3-4cm and it is embedded within 3-4 cm from the perineum position where the probe transmitter device 2 is placed when patient is sitting on the bed-type support 6 with legs opened to favour the minimum distance between prostate and probe transmitter device 2.

More specifically, "near-field" coupling means that the distance between the radiating antenna (e.g. the probe transmitter device 2) and the target is shorter than the wavelength assumed by the radiation in the patient body. Particularly, this distance can be lower than 1/5 of the wavelength, preferably lower than 1/10 of the wavelength assumed in the body or even less As an example, in prostate inspection the distance between the radiating antenna (e.g. the probe transmitter device 2) and the target is included in the range 3/25 - 4/19 of the wavelength evaluated in the patient body. Near-field operation enables a coupling with malignant tissues that is much higher than those normally achievable by conventional far-field systems used for microwave imaging.

The cancerous cells in depth tissues of the tumor region are excited from radio-frequency, that in turn generates a secondary electromagnetic field (i.e. an induced signal) for the large permittivity values of tumor tissues. The incident electromagnetic field and the induced electromagnetic field generate a scattered electromagnetic field (scattered signal). Particularly, since the tumor is in close distance from the probe transmitter device 2 in term of wavelength, the scattered field is a complex combination of incident field and the one generated by heterogeneous tissues with tumor (if present).

The closer is the probe transmitter device 2 to the tumor embedded into heterogeneous tissues, and the stronger is the coupling with the tumor, this in turn generates a more complex backscattering pattern with multiple scattering nulls.

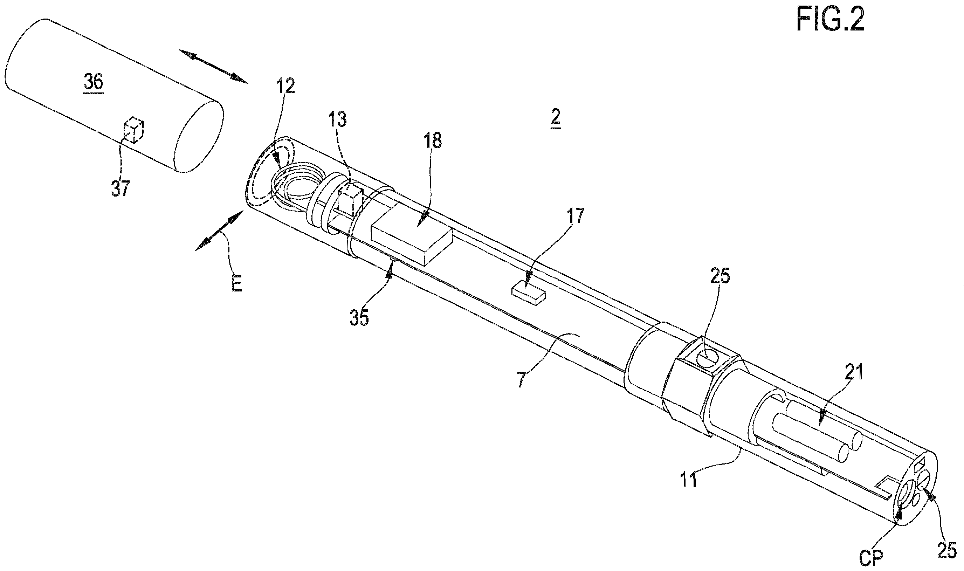

A particular embodiment of the probe transmitter device 2 is now described with reference to figure 2. The probe transmitter device 2 is provided with an antenna radiating element 12 structured to radiate a radio-frequency signal S_{RAD} which is a linearly polarized electromagnetic field. Particularly, the antenna radiating element 12 irradiates a non-ionizing radiofrequency radiation.

Moreover, the probe transmitter device 2 comprises an actuator 13 mechanically coupled to the antenna radiating element 12 to cause a rotation of the antenna radiating element so as to rotate the linear polarization of the radio-frequency signal S_{RAD}. The direction of the linear polarization is indicated with the letter E in figure 1.

In greater detail, probe transmitter device 2 comprises an external case 11 housing the antenna radiating element 12 and the actuator 13. The external case 11 is electromagnetically transparent to avoid any remarkable interaction with the waves from the antenna radiating element 12, or it is dielectric with permittivity comparable to the antenna radiating element 12, or with permittivity comparable with the normal tissues.

Particularly, the antenna radiating element 12 is a directive antenna. Preferably, the antenna radiating element 12 shows a radiation pattern including a main-lobe having an angular width comprised between 110° and 150° or, more preferably, equal to 120°.

In accordance with an example, the antenna radiating element 12 includes a two-ring antenna. Alternatively to the two-ring antenna a patch antenna can be employed.

Particularly, the linear polarization E of the radio-frequency signal S_{RAD} irradiated by the two-ring antenna 12 is directed perpendicularly to the longitudinal axis of the probe transmitter device 2 and lies in a plane comprising the two rings of the antenna.

The actuator 13 may comprise a motor and can be, preferably, a servomechanism provided with an electric motor, a position feedback potentiometer, a reduction gear and an actuator arm (not shown). Particularly, the servomechanism 13 is a digital servomechanism which can be controlled by digital signals. Preferably, the actuator 13 is housed in a container coated by a copper coating in order to provide a ground effect for the antenna radiating element 12 and a shield effect against electromagnetic interferences.

According to a particular embodiment, the actuator 13 is configured to rotate the antenna radiating element 12 in both rotation directions (clockwise or counter clockwise) of a pre-established angle. Preferably the maximum rotation angle is comprised between 60° and 180°, particularly, comprised between 90° to 180°. According to a more preferred solution, the antenna is rotated from 0° to 180°.

The rotation of the antenna radiating element 12 and consequently of the polarization E associated with the radio-frequency signal S_{RAD} allows triggering the interference and scattering phenomena that allows to obtain the anomaly detection.

The actuator 13 is configured to perform a rotation of the antenna radiating element 12 with an angular velocity equal or lower than 0.25 revolution per minute.

It is also observed that the patient positioning structure 1 is advantageously made of dielectric materials with minimal (ideally, null) dielectric contrast with respect of the external case 11 of the probe transmitter device 2. As an example, the patient positioning structure 1 is manufactured without using metallic elements having sizes that could cause reflections of the radiation emitted by the antenna radiating element 12 or scatter disturbances. Particularly, the size of possibly employed metallic elements is lower than λ/8, where λ is the wavelength of the radiation emitted by the probe transmitter device 2.

As shown in figure 2, the probe transmitter device 2 can be also provided with a probe controller 17 (CNT) and a transmitter module 18.

The probe controller 17 is configured to monitor and control some of the modules/components included in the probe transmitter device 2 and receive/send data and command signals from/to the external controller module 14. The probe controller 17 can be a microcontroller. The controller module 14 is, as an example, a Personal Computer PC.

The transmitter module 18 is configured to generate the radio-frequency signal S_{RAD} with a fixed frequency. As an example, the transmitter module 18 generates a radiation having frequency included into the range 1 MHz - 1000 MHz and particularly, 1 MHz - 900 MHz and more particularly, 300 MHz - 700 MHz.

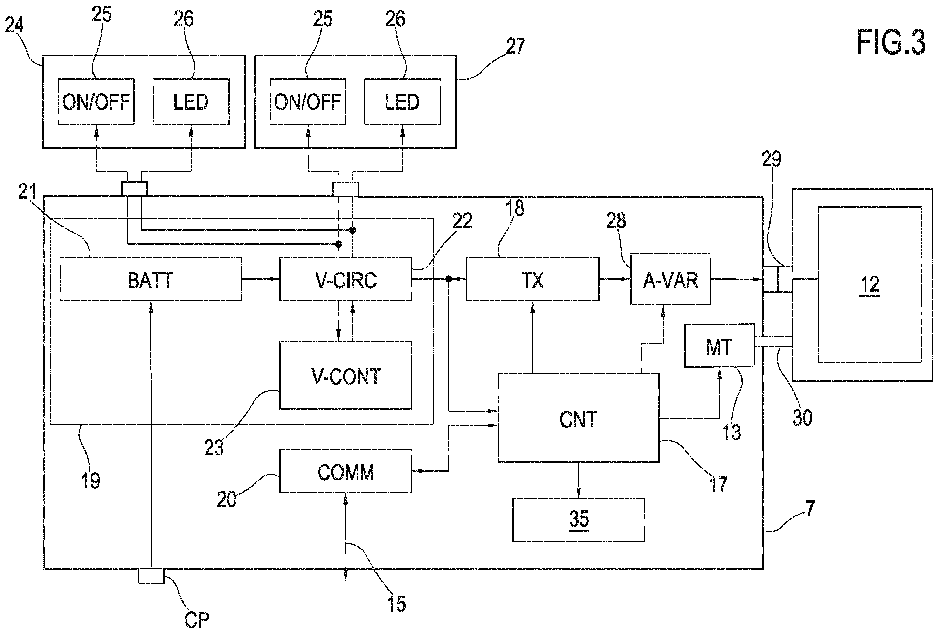

Figure 3 shows by functional modules a schematization of the components included in the probe transmitter device 2, in accordance with a specific embodiment. The probe transmitter device 2 as illustrated in figure 3 comprises an electronic board 7 connected to the antenna 12 and carrying the actuator 13 (MT), the probe controller 17 (CNT) and the transmitter module 18 (TX).

Moreover, according to an example, the electronic board 7 also includes a supply module 19 and a communication module 20 (COMM).

The supply module 19 comprises one or more batteries 21 (BATT), preferably, rechargeable batteries. As an example, four nickel-metal hydride batteries 1000 mAh can be employed. The supply module 19 also comprises a supply and turn-on circuit 22 (V-CIRC) fed by the batteries 21. The batteries 21 are connected with a charge connector CP for a charge cable.

Preferably, a supply control circuit 23 (V-CONT) is connected to the supply and turn-on circuit 22 and is configured to control the voltage level of the batteries 21 and the turn-off or turn-on of the probe transmitter device 2.

In accordance with a particular embodiment, the probe transmitter device 2 is also provided with a first interface module 24, connected to the supply and turn-on circuit 22, having an ON/OFF button 25 and a LED indicator 26 configured to show the status of the probe transmitter device 2.

Preferably, the probe transmitter device 2 is also provided with a second interface module 27, analogous to the first interface module 24. The first interface module 24 and the second interface module 27 are placed in different positions on the probe transmitter device 2 allowing the operator to choose the most convenient interface to employ depending on the particular orientation of probe transmitter device 2.

The communication module 20, connected to the probe controller 17, ensures telecommunications with the controller module 14 (figure 1) according to, preferably, a wireless technology, such as an example, the Bluetooth standard.

The transmitter module 18 is configured, as an example, to operate at a frequency of 433.9 MHz and produce an electric signal having a power of 100 mW. Advantageously, the transmitter module 18 is also provided with a feedback circuit (such as, a Phase-Locked Loop) in order to generate an electric signal having a stable frequency.

An output of the transmitter module 18 is, preferably, connected to an amplitude variation module 28 (A-VAR) which allows variating the amplitude level of the electrical signal generated by the transmitter module 18. According to an example, amplitude variation module 28 is an attenuator. According to a particular example, the amplitude variation module 28 can be an amplitude modulator configured to modulate the amplitude of the electrical signal generated by the transmitter module 18 and representing a carrier signal according to a sinusoidal modulating signal having, as an example, a frequency of 1 - 100 Hz.

An output of the amplitude variation module 28 (A-VAR) is connected to the antenna radiating element 12 *via* electric terminals 29 which allow maintaining electrical contact during rotation of the antenna radiating element 12. Particularly, the electric terminals 29 are brush contacts provided with linchpins having internal springs.

The antenna radiating element 12 is supported by means of a supporting structure 30 only schematized in figure 3.

Particularly, the probe controller 17 exchanges data and command signals with the controller module 14, *via* the communication module 20, and controls the transmitter module 18, the amplitude variation module 28 and the actuator 13.

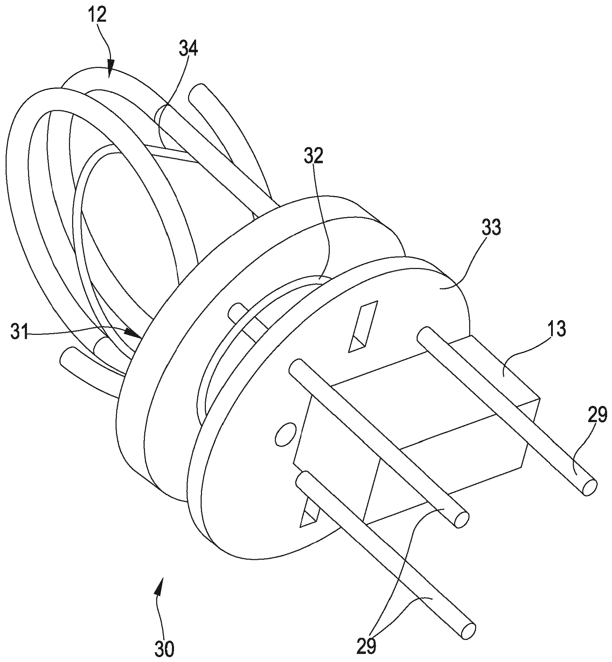

Figure 4 shows a particular embodiment of an assembly comprising the antenna radiating element 12, the actuator 13 (particularly, a servomechanism), the electric terminals 29 and the supporting structure 30.

Particularly, the antenna radiating element 12 shown in figure 4 is a double-ring antenna. The supporting structure 30 includes a rotatable base 31 (particularly, a disk) to which the antenna radiating element 12 is fixed. The rotatable base 31 is mechanically connected to the servomechanism 13.

The rotatable base 31 is made of a conductive material and is provided with a slither path 32 that can be contacted by the ends of the electric terminals 29. Particularly, the rotatable base 31 is a printed circuit board which can be made of polychlorinated biphenyl and coated with copper coating and an additional gold coating which is in contact with the electric terminals 29.

The electric terminals 29 are, as an examples, under the form of rods connected to the output of the transmitter module 18 (or the amplitude variation module 28) and configured to brush with the respective ends, which are under the pressure of the respective internal springs, against the slither path 32. Particularly, three electric terminals 29 are employed: two terminals 29 operate as electrical ground and the other terminal 29 carries the electrical signal. The use of three terminals 29 allows uniformly distributing the pressure exerted on the rotatable base 31.

According to the disclosed example, the coupling structure 30 also comprises a fixed base 33 (particularly, a disk) on which the servomechanism 13 is mounted. This fixed base 33 is provided with holes for the passage of the electric terminals 29 and can be a circuit printed board made of polychlorinated biphenyl and coated with copper.

It is observed that, advantageously, the rotatable base 31 is configured to provide a ground effect for the double-ring antenna 12 that allows increasing its directivity. Moreover, on the rotatable base 31 an additional component can be added to further increase the directivity of the double-ring antenna 12.

In accordance with the embodiment shown in figure 4, a matching component 34 (such as a metallic wire) has been fixed to the rotatable base 31 to achieve an impedance matching between the transmitter module 18 and the two-ring antenna 12.

As shown in the example of figure 1, the receiving device 4 is positioned under the bed type support 6, preferably on the floor. The detection apparatus 1000 can be also provided with at least one reflecting wall 3 positioned so as to reflect at least part of the incident radio-frequency signal S_{RAD} produced by the probe transmitter device 2, preferably not irradiated to the patient tissue, so as providing a reflected electromagnetic signal S_{R} for interferometry measurements. The reflecting wall 3 is positioned in such a way to avoid that it shields or obstructs the line of sight propagation from the probe transmitter device 2 to the receiving device 4.

According to a preferred example, particularly suitable for the embodiment of figure 1, four reflecting walls 3 are employed: each of the reflecting walls 3 are positioned orthogonally to the floor and so as to form a reflection structure surrounding the receiving device 4. The reflecting walls 3 are made, as an example, of a polyethylene board coated, on both sides, with aluminium providing reflecting and shielding effects. Particularly, the reflecting walls 3 extend from the floor to the bed type support 6. Preferably, with reference to the frequency of radio-frequency signal S_{RAD}, the reflector wall/s 3 show/s a reflectivity R whose amplitude is in the range between 0.6 and 1, more preferably, close to 1.

A particular embodiment of the receiving device 4 is shown in figure 1A. Accordingly, the receiving device 4 of figure 1A is provided with an array 38 comprising, as an example, at least two receiving antennas R1 and R2. The two antennas R1 and R2 are, as an example, dipoles on printed circuits. As another embodiment, the array 38 can include one or two sub-arrays each having eight antennas. According to a further embodiment, the array 38 is formed by six antennas.

The array 38 allows providing an electrical signal corresponding to a resulting electromagnetic signal S_{IN} obtained from a resulting backscattered electromagnetic signal S_{BS}, as backscattered by the irradiated patient tissue, and the reflected electromagnetic signal S_{R} as reflected by the reflecting walls 3.

The antennas included in the array 38 can be arranged horizontally, i.e. parallel to the floor.

The receiving device 4 also includes a receiver module 39 (RX) configured to treat the electrical signals provided by the array 38 (e.g. to perform filtering and amplification steps) and produce received electrical signals V_{REC} to be supplied to the processing module 5. Particularly, the receiver module 39 is configured to produce a plurality of received electrical signals V_{REC} each corresponding to a signal received at a different antenna (R1 or R2) of the array 38.

In the particular case in that the amplitude variation module 28 (Figure 3) is structured to operate as amplitude modulator, the receiving device 4 includes an amplitude demodulator (not shown) configured to obtain from the the electrical signals provided by the array 38 the electrical signals V_{REC} corresponding to the carrier signals as received at the array 38. It is observed that the amplitude modulation performed by the probe transmitter device 2 gives a further contribution to the trigger of the interference and scattering phenomena that allows obtaining the anomaly detection.

The processing module 5 can be structured to process the plurality of received electrical signals V_{REC} by applying a processing algorithm. Particularly, the processing algorithm is configured to analyse the trend of the power of the received electrical signals V_{REC} versus the corresponding time, for each antenna of the employed array 38, in order to determine the presence of nulls or minima or values below a threshold. The power of a received electrical signal V_{REC} depends on the power of the received electromagnetic field at the corresponding antenna of the array 38. The detection of nulls or minima or values below a threshold of the power indicates that there is an in-homogeneity such as tumor tissues.

Particularly, the processing unit 5 allows running the algorithm to localize the scattering nulls or minima in the pattern and provide an indication to the user (as an example, on the display 16) who is informed that the probe transmitter device 2 is in contact with a region of the patient's body wherein there is an in-homogeneity such as tumor tissues.

The controller module 14 is also configured to control the operation of the receiving module 4 via the link 15.

### Example of operation

In operation, with reference to the prostatic cancer, the cervical cancer or the rectum cancer, the patient is sitting on the bed type support 6 (in a gynaecological position) with the probe transmitter device 2 in close contact with the patient body (e.g., low buttocks and perineum), thanks to the motion of the slide 10. With reference to the diagnosis of other types of anomalies, the above described articulated arm can be employed to suitably contact the patient body.

The radio-frequency signal S_{RAD} radiated by the probe transmitter device 2 irradiates the tissue of the patient, in near-field coupling with tissues.

During the generation of the radio-frequency signal S_{RAD} the antenna radiating element 12 is rotated inside the external case 11 by the actuator 13 which receives commands from the probe controller 17. As an example, the rotation of the antenna radiating element 12 is performed continuously or by steps corresponding to small angles, i.e. less than 1°. The external case 11 is maintained stationary during the rotation of antenna radiating element 12. The rotation of the antenna radiating element 12 causes a corresponding rotation of the linear polarization E associated with the radio-frequency signal S_{RAD}.

This rotation is performed, with a fixed angular velocity, in a rotation direction until a pre-established maximum angle (i.e. 90° or 180 °) is reached and thus the rotation is carried out in the opposite direction. During rotation, the brush-type terminal 29 ensures that the antenna radiating element 12 is fed with the electrical signal produced by the transmitter module 18.

Moreover, it's to be noted that the rotation of the antenna radiating element 12 is activated by the clinician by the controller module 14, which is connected to the probe controller 17 *via* the preferred wireless link 15 and is carried out automatically. As an example, a 180° rotation is performed in 120 seconds.

The resulting backscattered electromagnetic signal S_{BS} emerges from the patient tissue and interferes with the reflected electromagnetic signals S_{R}: so producing the resulting electromagnetic signal S_{IN} which reaches the array 38 of the receiving device 4. The electrical signal provided by the array 38 is processed by the receiver module 39 which produces the corresponding electrical signal V_{REC}.

The processing module 5 analyses the electrical signal V_{REC} and (e.g. after an analog-to-digital conversion) obtains data from which detects corresponding null and minima, if any, and such results are shown on the display 16. The processing module 5 analyses the electrical signals V_{REC} as obtained during an operation interval including the rotation made.

The irradiation step, performed together with the rotation of the antenna radiating element 12, is, as an example, carried out until an anomaly is detected or a pre-established time is reached.

It is noticed that the Applicant has verified that the above described controlled rotation of the antenna radiating element 12 and of the linear polarization E of radio-frequency signal S_{RAD} allows producing with a particular efficiency the phenomena from which the minima or null can be detected.

The controller module 14 can report both the positions assumed by the antenna radiating 12 and the measurements obtained from the array 38, so as to guarantee the repetition of the diagnostic test over multiple diagnostic sessions separated several weeks one another to diagnoses any change in tissues or the evolution of disease.

Particularly, the processing module 5 can be configured to map the position of the rotation angle assumed by the antenna radiating element 12 obtained from the control unit 14 with the corresponding nulls or minima. Moreover, the processing module 5 can indicate for which antenna of the array 38 the null or minimum has been detected.

### Further embodiment

In accordance with a particular embodiment, as shown in figures 2 and 3, the electronic board 7 is also provided with a position sensor 35. According to this embodiment, the probe transmitter device 2 comprises a sliding lid 36 to be put on an end of the probe transmitter device 2 provided with the antenna radiating element. The sliding lid 36 carries a magnet 37 which can be detected by the position sensor 35 when it is in proximity of the sensor itself. The sliding lid 36 can be manufactured using the same material of the external case 11.

In operation, if the sliding lid 36 is properly pushed against the patient body, the magnet 37 is detected by the position sensor 35 which generates a detection signal that allows the probe controller 17 recognizing a correct operative configuration and so enabling the irradiation of the radio-frequency signal S_{RAD}.

On the contrary, if the probe transmitter device 2 and the sliding lid 36 are not sufficiently pushed on the patient body, the magnet 37 is not in proximity of the position sensor 35: in this situation the position sensor 35 generates another detection signal that allows the probe controller 17 recognizing a non-correct operative configuration, so disabling/preventing the irradiation of the radio-frequency signal S_{RAD}.

It is observed that the detection apparatus 1000 provided with the described probe transmitter device 2 is less complex and cumbersome of the known apparatus and allows obtaining high detection performances. Indeed, the Applicant has noticed that the described rotation of the liner polarization of the radio-frequency signal is an efficient manner to generate the phenomena employed to detect the anomalies.

Moreover, the Applicant observes that the described apparatus can be realized avoiding that the clinician holds the probe transmitter device, so eliminating the coupling between the clinician and the probe, coupling itself that might generate false nulls or minima with erroneous diagnosis.

In addition, the detection apparatus 1000 above described may guarantee fixed reciprocal positions among the probe transmitter device 2, the patient and the array 38.

The described detection apparatus 1000 offers an automatic implementation of the detection steps and avoids complex motion of the probe transmitter device 2 during irradiation.

### List of references employed in the figures:

- detection apparatus 1000
- patient positioning structure 1
- probe transmitter device 2
- reflecting wall 3
- receiving device 4
- processing module 5 (PROC)
- bed type support 6
- electronic board 7
- mounting frame 8
- base 9
- slide 10
- external case 11
- antenna radiating element 12
- actuator 13
- controller module 14 (CONTR)
- wired or wireless link 15
- display 16 (DISP)
- probe controller 17 (CNT)
- transmitter module 18
- supply module 19
- communication module 20 (COMM)
- batteries 21 (BATT)
- supply and turn-on circuit 22 (V-CIRC)
- supply control circuit 23 (V-CONT)
- first interface module 24
- ON/OFF button 25
- LED indicator 26
- second interface module 27
- amplitude variation module 28
- electric terminals 29
- coupling structure 30
- rotatable base 31
- fixed base 33
- matching element 34
- position sensor 35
- sliding lid 36
- magnet 37
- array 38
- receiver module 39
- charge connector CP
- linear polarization E
- radio-frequency signal S_{RAD}
- resulting electromagnetic signal S_{IN}
- resulting backscattered electromagnetic signal S_{BS}
- reflected electromagnetic signal S_{R}
- electrical signals V_{REC}

## Claims

1. A tissue anomaly detection system (1000), comprising:
a probe transmitter device (2) structured to radiate a radio-frequency signal (S_{RAD}) to irradiate a patient tissue in near-field conditions so as to produce a resulting radio-frequency signal (S_{IN}) and comprising: an antenna radiating element (12) configured to radiate the radio-frequency signal (S_{RAD}) with a linear polarization (E);
a receiving device (4) structured to receive the resulting signal (S_{IN}) and provide a corresponding received electrical signal (V_{REC});
a processing module (5) configured to process the received electrical signal (V_{REC}) and provide an information indicating detection of an anomaly in said tissue; wherein the processing module (5) is configured to: analyse the trend of a power associated to the received electrical signal (V_{REC}) versus time and detect said anomaly associated with nulls or minima or values under a threshold in said trend of the power;
**characterized in that** the transmitter device (2) further includes an actuator device (13) mechanically coupled to the antenna radiating element (12) and configured to cause a rotation of the antenna radiating element (12) so as to rotate the polarization (E) of the radio-frequency signal (S_{RAD}) and triggering interference and scattering phenomena that allow obtaining the anomaly detection.

2. The system (1000) of claim 1, wherein the probe transmitter device (2) has a single antenna radiating element (12) configured to radiate the radio-frequency signal (S_{RAD}) with the linear polarization (E) .

3. The system (1000) of claim 1 or 2, wherein the actuator is configured to cause a rotation of the antenna radiating element (12) of a maximum rotation angle comprised between 60° and 180°, particularly, comprised between 90° to 180°

4. The system (1000) of claim 1, wherein the apparatus further includes:
a control module (14; 17) configured to send control signals to the actuator (13) to perform a rotation of the antenna radiating element (12) and of the linear polarization (E) according to a pre-established angle.

5. The system (1000) according to at least one of the preceding claims, wherein the probe transmitter device (2) further comprises:
at least one brush-type terminal (29) configured to electrically contact the antenna radiating element (12) and carry an electrical signal corresponding to said radio-frequency signal (S_{RAD}) to be irradiated.

6. The system (1000) according to at least claim 5, wherein said the probe transmitter device (2) comprises:
a supporting structure (30) on which the antenna radiating element (12) is mounted; the supporting structure (30) being coupled to said actuator (13) to allow the rotation of the antenna radiating element (12) .

7. The system (1000) according to claim 6, wherein said supporting structure (30) comprises:
a rotatable base (31) coupled to the actuator (13) and to which the antenna radiating element (12) is fixed and electrically connected; said rotatable base (31) being contacted by the least one brush-type terminal (29) ;
a fixed support base (33) on which the actuator is mounted.

8. The system (1000) according to claim 6, wherein said actuator (13) comprises a servomechanism.

9. The system (1000) according to at least one of the preceding claims, wherein said antenna radiating element (12) is a directive antenna.

10. The system (1000) of claim 9, wherein said directive antenna shows a radiation pattern including a main-lobe having an angular width comprised between 110° and 150°.

11. The system (1000) of at least one of the preceding claims, wherein the antenna radiating element (12) includes a two-rinq antenna.

12. The system (1000) of at least claims 7 and 10, wherein said rotatable base (31) further includes components (31) configured to increase the directivity of the antenna radiating element (12).

13. The system (1000) according to at least one of the preceding claims, wherein the probe transmitter device (2) comprises:
a transmitter module (18) configured to provide a generated electrical signal at a fixed frequency corresponding to said radio-frequency signal (S_{RAD}) to be irradiated; the transmitter module (18) being connected to said antenna radiating element (12).

14. The system (1000) according to at least one of the preceding claims, wherein:
the detection system (1000) further comprises an external control module (14) which is configured to exchange/data command with the probe transmitter device; and
the probe transmitter device (2) further comprises a communication module (20) configured to exchange data and/or commands with the external control module (14).

15. The system (1000) according to at least claim 4, wherein the probe transmitter device (2) includes:
an external case (11) including the antenna radiating element (12) and the actuator (13);
a position sensor (35) mounted on the external case (11) ;
a sliding structure (36) configured to be mounted on the external case (11) and to be put in contact with the patient tissue; the sliding structure (36) is configured to assume a pushed configuration and an non-pushed configuration depending on a pressure exerted on the patient tissue;
a magnet (37) fixed to the sliding structure(36) in such a way that the magnet is in proximity of the position sensor in the pushed configuration and is not in proximity of the position sensor in the non-pushed configuration;
wherein said position sensor (35) is adapted to produce a detection signal depending on the position of said magnet with respect to the position sensor and said control module (14, 17) is configured to enable/disable the irradiation of the radio-frequency signal on the base of said detection signal produced by the position sensor (35).

16. The system (1000) of at least claim 1, further comprising:
a patient positioning structure (1) defining a positioning plane (6) for the patient,
and wherein:
the probe transmitter device (2) is positioned at a side with respect to the positioning plane (6) and the receiving device (4) is positioned at an opposite side with respect to the positioning plane; and
the apparatus (1000) comprises at least one reflecting wall (3) structured to reflect said radio-frequency signal (S_{RAD}).

17. The system (1000) of at least claim 15, wherein a mounting frame (8) is configured to move the probe transmitter device (2) to selectively assume the pushed configuration and the non-pushed configuration.

18. The system (1000) of claim 17, wherein said mounting frame (8) comprises a slide supporting the probe transmitter device (2) or an articulated arm supporting the probe transmitter device (2).

## Patentansprüche

1. Ein System zur Erkennung von Gewebeanomalien (1000), umfassend:
eine Sondensendevorrichtung (2), die so strukturiert ist, dass sie ein Hochfrequenzsignal (S_{RAD}) ausstrahlt, um ein Patientengewebe unter Nahfeldbedingungen zu bestrahlen, um ein resultierendes Hochfrequenzsignal (S_{IN}) zu erzeugen, und umfassend: ein Antennenausstrahlungselement (12) konfiguriert, um das Hochfrequenzsignal (S_{RAD}) mit einer linearen Polarisation (E) auszustrahlen;
eine Empfangsvorrichtung (4), die so strukturiert ist, dass sie das resultierende Signal (S_{IN}) empfängt und ein entsprechendes empfangenes elektrisches Signal (V_{REC}) bereitstellt;
ein Verarbeitungsmodul (5), das konfiguriert ist, um das empfangene elektrische Signal (V_{REC}) zu verarbeiten und eine Information bereitzustellen, die die Erkennung einer Anomalie in dem Gewebe anzeigt; wobei das Verarbeitungsmodul (5) konfiguriert ist, um: den Trend einer Leistung, die dem empfangenen elektrischen Signal (V_{REC}) zugeordnet ist, über die Zeit zu analysieren und die Anomalie zu erfassen, die mit Nullen oder Minima oder Werten unter einem Schwellenwert in dem Trend der Leistung verbunden ist;
**dadurch gekennzeichnet, dass** die Sendevorrichtung (2) ferner eine Aktuatorvorrichtung (13) enthält, die mechanisch mit dem Antennenausstrahlungselement (12) gekoppelt und konfiguriert ist, um eine Drehung des Antennenausstrahlungselements (12) zu bewirken, um die Polarisation (E) des Hochfrequenzsignals (S_{RAD}) zu drehen und Interferenz- und Streuphänomene auszulösen, die es ermöglichen, die Anomalieerkennung zu erhalten.

2. System (1000) nach Anspruch 1, wobei die Sondensendevorrichtung (2) ein einzelnes Antennenausstrahlungselement (12) aufweist, das konfiguriert ist, um das Hochfrequenzsignal (S_{RAD}) mit der linearen Polarisation (E) auszustrahlen.

3. Das System (1000) nach Anspruch 1 oder 2, wobei der Aktuator konfiguriert ist, um eine Drehung des Antennenausstrahlungselements (12) um einen maximalen Drehwinkel zwischen 60° und 180°, insbesondere zwischen 90° bis 180° zu veranlassen.

4. Das System (1000) nach Anspruch 1, wobei die Vorrichtung ferner umfasst:
ein Steuermodul (14; 17), das konfiguriert ist, um Steuersignale an den Aktuator (13) zu senden, um eine Drehung des Antennenausstrahlungselements (12) und der linearen Polarisation (E) gemäß einem vorbestimmten Winkel durchzuführen.

5. System (1000) nach mindestens einem der vorhergehenden Ansprüche, wobei die Sondensendevorrichtung (2) ferner umfasst:
mindestens einen bürstenartigen Anschluss (29), der konfiguriert ist, um das Antennenausstrahlungselement (12) elektrisch zu kontaktieren und ein elektrisches Signal zu führen, das dem auszustrahlenden Hochfrequenzsignal (S_{RAD}) entspricht.

6. System (1000) nach mindestens Anspruch 5, wobei die Sondensendevorrichtung (2) umfasst:
eine Stützstruktur (30), auf der das Antennenausstrahlungselement (12) montiert ist; wobei die Stützstruktur (30) mit dem Aktuator (13) gekoppelt ist, um die Drehung des Antennenausstrahlungselements (12) zu ermöglichen.

7. System (1000) nach Anspruch 6, wobei die Stützstruktur (30) umfasst:
eine drehbare Basis (31), die mit dem Aktuator (13) gekoppelt ist und an der das Antennenausstrahlungselement (12) befestigt und mit der es elektrisch verbunden ist; wobei die drehbare Basis (31) von dem mindestens einen bürstenartigen Anschluss (29) kontaktiert wird;
eine feste Stützbasis (33), auf welcher der Aktuator montiert ist.

8. Das System (1000) nach Anspruch 6, wobei der Aktuator (13) einen Servomechanismus umfasst.

9. Das System (1000) nach mindestens einem der vorhergehenden Ansprüche, wobei das Antennenausstrahlungselement (12) eine Richtantenne ist.

10. Das System (1000) nach Anspruch 9, wobei die Richtantenne ein Strahlungsmuster mit einer Hauptkeule mit einer Winkelbreite zwischen 110° und 150° aufweist.

11. Das System (1000) nach zumindest einem der vorhergehenden Ansprüche, wobei das Antennenausstrahlungselement (12) eine Zwei-Ring-Antenne umfasst.

12. Das System (1000) nach mindestens den Ansprüchen 7 und 10, wobei die drehbare Basis (31) ferner Bauelemente (31) umfasst, die ausgelegt sind, um die Richtwirkung des Antennenausstrahlungselements (12) zu erhöhen.

13. System (1000) nach mindestens einem der vorhergehenden Ansprüche, wobei die Sondensendevorrichtung (2) umfasst:
ein Sendemodul (18), das konfiguriert ist, um ein erzeugtes elektrisches Signal mit einer festen Frequenz bereitzustellen, die dem auszustrahlenden Hochfrequenzsignal (S_{RAD}) entspricht; wobei das Sendemodul (18) mit dem Antennenausstrahlungselement (12) verbunden ist.

14. System (1000) nach mindestens einem der vorhergehenden Ansprüche, wobei:
das Erkennungssystem (1000) ferner ein externes Steuermodul (14) umfasst, das zum Austausch von Daten/Befehlen mit der Sondensendevorrichtung konfiguriert ist; und
die Sondensendevorrichtung (2) ferner ein Kommunikationsmodul (20) umfasst, das konfiguriert ist, um Daten und/oder Befehle mit dem externen Steuermodul (14) auszutauschen.

15. System (1000) nach mindestens Anspruch 4, wobei die Sondensendevorrichtung (2) umfasst:
ein äußeres Gehäuse (11) mit dem Antennenausstrahlungselement (12) und dem Aktuator (13);
einen Positionssensor (35), der an dem äußeren Gehäuse (11) angebracht ist;
eine Gleitstruktur (36), die konfiguriert ist, um an dem äußeren Gehäuse (11) angebracht zu werden und mit dem Patientengewebe in Kontakt gebracht zu werden; wobei die Gleitstruktur (36) so konfiguriert ist, dass sie abhängig von einem auf das Patientengewebe ausgeübten Druck eine Druckkonfiguration und eine nicht Druckkonfiguration annimmt.
einen Magneten (37), der an der Gleitstruktur (36) derart befestigt ist, dass sich der Magnet in der gedrückten Konfiguration in der Nähe des Positionssensors befindet und sich in der nicht gedrückten Konfiguration nicht in der Nähe des Positionssensors befindet;
wobei der Positionssensor (35) angepasst ist, um ein Erkennungssignal in Abhängigkeit von der Position des Magneten in Bezug auf den Positionssensor zu erzeugen, und das Steuermodul (14, 17) konfiguriert ist, um die Ausstrahlung des Hochfrequenzsignals auf der Basis des vom Positionssensor (35) erzeugten Erkennungssignals zu aktivieren/deaktivieren.

16. System (1000) nach mindestens Anspruch 1, ferner umfassend:
eine Patientenpositionierungsstruktur (1), die eine Positionierungsebene (6) für den Patienten definiert,
und worin:
die Sondensendevorrichtung (2) auf einer Seite in Bezug auf die Positionierebene (6) positioniert ist und die Empfangsvorrichtung (4) auf einer gegenüberliegenden Seite in Bezug auf die Positionierebene positioniert ist; und
die Vorrichtung (1000) mindestens eine reflektierende Wand (3) umfasst, die so strukturiert ist, dass sie das Hochfrequenzsignal (S_{RAD}) reflektiert.

17. Das System (1000) nach mindestens Anspruch 15, wobei der Montagerahmen (8) konfiguriert ist, um die Sondensendevorrichtung (2) zu bewegen, um selektiv die gedrückte Konfiguration und die nicht gedrückte Konfiguration anzunehmen.

18. Das System (1000) nach Anspruch 17, wobei der Montagerahmen (8) eine die Sondensendervorrichtung (2) unterstützende Gleitführung oder einen die Sondensendervorrichtung (2) unterstützenden Gelenkarm umfasst.

## Revendications

1. Système de détection d'anomalie de tissu (1000), comprenant :
un dispositif d'émetteur de sonde (2) structuré pour émettre un signal radiofréquence (S_{RAD}) afin d'irradier un tissu de patient dans des conditions en champ proche de façon à produire un signal radiofréquence résultant (S_{IN}) et comprenant : un élément rayonnant d'antenne (12) configuré pour émettre le signal radiofréquence (S_{RAD}) avec une polarisation linéaire (E) ;
un dispositif de réception (4) structuré pour recevoir le signal résultant (S_{IN}) et fournir un signal électrique reçu correspondant (V_{REC}) ;
un module de traitement (5) configuré pour traiter le signal électrique reçu (V_{REC}) et fournir des informations indiquant la détection d'une anomalie dans ledit tissu ; le module de traitement (5) étant configuré pour : analyser l'évolution d'une puissance associée au signal électrique reçu (V_{REC}) par rapport au temps et détecter ladite anomalie associée à des valeurs nulles ou à des valeurs minimales ou à des valeurs situées sous un seuil dans ladite évolution de la puissance ;
**caractérisé par le fait que** le dispositif d'émetteur (2) comprend en outre un dispositif d'actionneur (13) couplé mécaniquement à l'élément rayonnant d'antenne (12) et configuré pour entraîner une rotation de l'élément rayonnant d'antenne (12) de façon à tourner la polarisation (E) du signal radiofréquence (S_{RAD}), et une interférence de déclenchement et des phénomènes de diffusion qui permettent d'obtenir la détection d'anomalie.

2. Système (1000) selon la revendication 1, dans lequel le dispositif d'émetteur de sonde (2) possède un unique élément rayonnant d'antenne (12) configuré pour émettre le signal radiofréquence (S_{RAD}) avec la polarisation linéaire (E).

3. Système (1000) selon la revendication 1 ou 2, dans lequel l'actionneur est configuré pour entraîner une rotation de l'élément rayonnant d'antenne (12) d'un angle de rotation maximal compris entre 60° et 180°, en particulier, compris entre 90° et 180°.

4. Système (1000) selon la revendication 1, dans lequel l'appareil comprend en outre :
un module de commande (14 ; 17) configuré pour envoyer des signaux de commande à l'actionneur (13) afin de réaliser une rotation de l'élément rayonnant d'antenne (12) et de la polarisation linéaire (E) selon un angle préétabli.

5. Système (1000) selon au moins l'une des revendications précédentes, dans lequel le dispositif d'émetteur de sonde (2) comprend en outre :
au moins une borne de type balai (29) configurée pour entrer électriquement en contact avec l'élément rayonnant d'antenne (12) et transporter un signal électrique correspondant audit signal radiofréquence (S_{RAD}) à irradier.

6. Système (1000) selon au moins la revendication 5, dans lequel ledit dispositif d'émetteur de sonde (2) comprend :
une structure de support (30) sur laquelle l'élément rayonnant d'antenne (12) est monté ; la structure de support (30) étant couplée audit actionneur (13) pour permettre la rotation de l'élément rayonnant d'antenne (12) .

7. Système (1000) selon la revendication 6, dans lequel ladite structure de support (30) comprend :
une base rotative (31) couplée à l'actionneur (13) et sur laquelle l'élément rayonnant d'antenne (12) est fixé et relié électriquement ; ladite base rotative (31) étant mise en contact par l'au moins une borne de type balai (29) ;
une base de support fixe (33) sur laquelle l'actionneur est monté.

8. Système (1000) selon la revendication 6, dans lequel ledit actionneur (13) comprend un servo-mécanisme.

9. Système (1000) selon au moins l'une des revendications précédentes, dans lequel ledit élément rayonnant d'antenne (12) est une antenne directive.

10. Système (1000) selon la revendication 9, dans lequel ladite antenne directive présente un motif de rayonnement comprenant un lobe principal ayant une largeur angulaire comprise entre 110° et 150°.

11. Système (1000) selon au moins l'une des revendications précédentes, dans lequel l'élément rayonnant d'antenne (12) comprend une antenne à deux anneaux.

12. Système (1000) selon au moins les revendications 7 et 10, dans lequel ladite base rotative (31) comprend en outre des composants (31) configurés pour augmenter la directivité de l'élément rayonnant d'antenne (12) .

13. Système (1000) selon au moins l'une des revendications précédentes, dans lequel le dispositif d'émetteur de sonde (2) comprend :
un module d'émetteur (18) configuré pour fournir un signal électrique généré à une fréquence fixe correspondant audit signal radiofréquence (S_{RAD}) à irradier ; le module d'émetteur (18) étant relié audit élément rayonnant d'antenne (12).

14. Système (1000) selon au moins l'une des revendications précédentes, dans lequel :
le système de détection (1000) comprend en outre un module de commande externe (14) qui est configuré pour échanger des données/instructions avec le dispositif d'émetteur de sonde ; et
le dispositif d'émetteur de sonde (2) comprend en outre un module de communication (20) configuré pour échanger des données et/ou instructions avec le module de commande externe (14).

15. Système (1000) selon au moins la revendication 4, dans lequel le dispositif d'émetteur de sonde (2) comprend :
un boîtier externe (11) comprenant l'élément rayonnant d'antenne (12) et l'actionneur (13) ;
un capteur de position (35) monté sur le boîtier externe (11) ;
une structure de coulissement (36) configurée pour être montée sur le boîtier externe (11) et pour être mise en contact avec le tissu de patient ; la structure de coulissement (36) étant configurée pour adopter une configuration poussée et une configuration non poussée en fonction d'une pression appliquée sur le tissu de patient ;
un aimant (37) fixé à la structure de coulissement (36) de telle sorte que l'aimant est à proximité du capteur de position dans la configuration poussée et n'est pas à proximité du capteur de position dans la configuration non poussée ;
ledit capteur de position (35) étant conçu pour produire un signal de détection en fonction de la position dudit aimant par rapport au capteur de position, et ledit module de commande (14, 17) étant configuré pour activer/désactiver l'irradiation du signal radiofréquence sur la base dudit signal de détection produit par le capteur de position (35).

16. Système (1000) selon au moins la revendication 1, comprenant en outre :
une structure de positionnement de patient (1) définissant un plan de positionnement (6) pour le patient,
et dans lequel :
le dispositif d'émetteur de sonde (2) est positionné au niveau d'un côté par rapport au plan de positionnement (6), et le dispositif de réception (4) est positionné au niveau d'un côté opposé par rapport au plan de positionnement ; et
l'appareil (1000) comprend au moins une paroi réfléchissante (3) structurée pour réfléchir ledit signal radiofréquence (S_{RAD}).

17. Système (1000) selon au moins la revendication 15, dans lequel un cadre de montage (8) est configuré pour déplacer le dispositif d'émetteur de sonde (2) pour sélectivement adopter la configuration poussée et la configuration non poussée.

18. Système (1000) selon la revendication 17, dans lequel ledit cadre de montage (8) comprend un coulisseau portant le dispositif d'émetteur de sonde (2) ou un bras articulé portant le dispositif d'émetteur de sonde (2) .
